Europäisches Patentamt

European Patent Office

Office européen des brevets

(11) Publication number: **0 336 371**
**A2**

# EUROPEAN PATENT APPLICATION

(21) Application number: **89105887.7**

(22) Date of filing: **04.04.89**

(51) Int. Cl.⁴: **C07F 7/18**

(30) Priority: **04.04.88 US 177097**
**04.04.88 US 177151**
**04.04.88 US 177153**
**04.04.88 US 177152**
**20.05.88 US 196680**

(43) Date of publication of application:
**11.10.89 Bulletin 89/41**

(84) Designated Contracting States:
**AT BE CH DE ES FR GB GR IT LI LU NL SE**

(71) Applicant: **ETHYL CORPORATION**
**451 Florida Boulevard**
**Baton Rouge, LA 70801(US)**

(72) Inventor: **Stahly, Glenn Patrick**
**517 Corsair Drive**
**Baton Rouge Louisiana 70801(US)**

(74) Representative: **Dipl.-Ing. Schwabe, Dr. Dr. Sandmair, Dr. Marx**
**Stuntzstrasse 16**
**D-8000 München 80(DE)**

(54) **Gem-disubstituted cyclohexadienones and their production.**

(57) Gem-disubstituted cyclohexadienone in which the gem substituents are a perfluoroalkyl group and a trihydrocarbylsiloxy group are prepared by perfluoroalkylating a quinone with a perfluoroalkyltrihydrocarbylsilane in the presence of an active catalyst under essentially anhydrous conditions, preferably in a suitable liquid phase reaction medium, most preferably in a dipolar aprotic solvent. These gem-disubstituted compounds are readily convertible to perfluoroalkyl-substituted aromatics and thus provide a means of circumventing the traditional need for photochlorination followed by halogen exchange in the preparation of such products.

EP 0 336 371 A2

## GEM-DISUBSTITUTED CYCLOHEXADIENONES AND THEIR PRODUCTION

This invention relates in general to perfluoroalkyl aromatic compounds. More particularly, this invention relates to a new class of perfluoroalkyl substituted compounds from which perfluoroalkyl aromatic compounds can be readily produced and to novel methods by which such perfluoroalkyl substituted compounds may be prepared.

Perfluoroalkyl aromatic compounds such as benzotrifluoride, 4-chlorobenzotrifluoride and 3-aminobenzotrifluoride are used in the production of a variety of products such as pharmaceuticals, crop protection chemicals, germicides, dyes, and the like. The classical method of forming trifluoromethyl aromatics involves the photochemical side-chain chlorination of a methyl aromatic compound to form a perchloromethyl substituted aromatic which in turn is reacted with hydrogen fluoride to effect an exchange of fluorine atoms for the chlorine atoms on the methyl group. Ortho- and para-trifluoromethylphenols and anilines are even more difficult to make. They have been synthesized by photochemical side-chain chlorination or bromination of the appropriate nitrotoluene to form the perhalomethyl nitrobenzene. This product is treated with hydrogen fluoride to form the perfluoromethyl nitrobenzene, which is then reduced to the perfluoromethyl aniline. Diazotization and hydrolysis of the latter forms the perfluoromethyl phenol.

In Example 6 of U. S. Pat. No. 4,634,787, Wang reports that reaction between quinone and trichloromethyltrimethylsilane in tetrahydrofuran using tetrabutylammonium fluoride as catalyst yielded 4-(trichloromethyl)-4-(trimethylsilyloxy)-2,5-cyclohexadien-1-one. While the patentee refers to compounds having a -CX3 group in which each X is independently halo, according to the patentee: ". . . preferably, each X is independently chloro or bromo. More preferably, each X is the same and is chloro or bromo. Even more preferably, each X is chloro. Preferred silanes [used as reactants in the process] are trichloromethylsilanes and the most preferred silane is trichloromethyltrimethylsilane."

In accordance with this invention there is provided a new class of perfluoroalkyl substituted compounds from which a wide variety of perfluoroaromatic compounds can readily be prepared. In addition, this invention provides a novel catalytic process by which these new perfluoroalkyl substituted compounds can be prepared.

This invention is in part based on the discovery that quinones may be perfluoroalkylated by means of perfluoroalkyltrihydrocarbyl silane in the presence of certain catalysts, specifically (A) certain fluorine-free active alkali metal salts, (B) certain trivalent phosphorus compounds corresponding to the formula $R_3P$, wherein R is hydrocarbyloxy or dihydrocarbylamino, (C) quaternary ammonium bifluorides, quaternary phosphonium bifluorides and/or alkali metal bifluorides, (D) aminopyridine compounds, and (E) any of the aforementioned catalysts or an alkali metal fluoride or quaternary ammonium monofluoride together with a proton source.

The reaction results in the formation of gem-disubstituted cyclohexadienones in which the gem substituents are a perfluoroalkyl group and a trihydrocarbylsiloxy group. These gem-disubstituted compounds in turn can be readily converted to perfluoroalkyl substituted aromatics. Thus this invention circumvents the traditional need for photochlorination followed by halogen exchange using hydrogen fluoride as a means of preparing perfluoroalkyl aromatic compounds.

It is interesting to note that NaF and $CaF_2$, two of the fluoride ion catalysts recommended in U. S. 4,634,787 for use as catalysts are ineffective as catalysts in the perfluoroalkylation process of this invention.

The process of this invention is conducted under essentially anhydrous conditions, preferably in a suitable liquid phase reaction medium. The preferred solvents or liquid reaction media for use in the process are dipolar aprotic solvents such as N,N-dimethylformamide, N,N-diethylformamide, N,N-dimethylacetamide, sulfolane, acetonitrile, hexamethylphosphoramide, nitrobenzene, dimethylsulfoxide, N-methylpyrrolidone, and the like. When performing the reaction in a substantially anhydrous aprotic solvent of low polarity such as tetrahydrofuran, 1,4-dioxane or the like, it is sometimes desirable to utilize a phase transfer catalyst such as a crown ether. See in this connection C. M. Starks and C. Liott, Phase Transfer Catalysts, Academic Press, 1978.

A variety of alkali metal salt catalysts devoid of fluorine may be used in the practice of this invention provided they exhibit the appropriate catalytic activity. In this connection, not all fluorine-free alkali metal salts exhibit a catalytic effect in the reaction and thus in any given instance where the suitability of a given fluorine-free alkali metal salt is not known, recourse should be had to the simple expedient of performing a few pilot experiments to determine whether the material will serve as a catalyst in the reaction.

Alkali metal salts devoid of fluorine which are active catalysts in the process of this invention include the alkali metal azides such as lithium azide, sodium azide, and potassium azide; alkali metal cyanides such as sodium cyanide, potassium cyanide, and cesium cyanide; alkali metal hydroxides such as sodium hydroxide and potassium hydroxide; alkali metal carbonates such as potassium carbonate and cesium carbonate; and the like.

It is not known how or why the catalysts function in the process of this invention. Nor, is the structure or composition of the actual catalytic species known. All that is known is that when the catalyst is added to the reaction system in the form of an inorganic salt such as above-described, preferably in finely divided form, the reaction proceeds. In the absence of the catalyst, no reaction occurs.

The most preferred of the alkali metal salt catalysts are potassium cyanide and potassium carbonate, because of their high activity, ready availability, and low cost. Reactions performed in acetonitrile using potassium cyanide or potassium carbonate as the catalyst and in dimethylformamide with potassium carbonate as the catalyst have been found particularly efficacious.

A wide variety of trihydrocarbylphosphites and hexahydrocarbyl phosphorous triamides can be used as catalysts in the process of this invention. Illustrative trihydrocarbylphosphites include trimethylphosphite, triethylphosphite, tripropylphosphite, triisopropylphosphite, tributylphosphite, tridodecylphosphite, triallylphosphite, trioleylphosphite, tricyclohexylphosphite, tricyclopropylcarbinylphosphite, triphenylphosphite, tritolylphosphite, tribenzylphosphite, phenyldiethylphosphite, dibenzyloctadecylphosphite, and the like. Among the hexahydrocarbylphosphorous triamides that may be employed as catalysts are hexamethylphosphorous triamide, hexaethylphosphorous triamide, hexapropylphosphorous triamide, hexaisopropylphosphorous triamide, hexabutylphosphorous triamide, hexacyclopentylphosphorous triamide, hexaphenylphosphorous triamide, hexa(4-ethylphenyl)phosphorous triamide, hexa(2-phenethyl)phosphorous triamide, hexacrotonylphosphorous triamide, and the like.

A mixture of two or more trihydrocarbylphosphites or of two or more hexahydrocarbylphosphorous triamides may be used as the catalyst. Likewise, mixtures of one or more trihydrocarbylphosphites with one or more hexahydrocarbylphosphorous triamides can be used for this purpose, if desired.

It is not known how or why the catalysts function in the process of this invention. Nor, is the structure or composition of the actual catalytic species known. All that is known is that when the catalyst is added to the reaction system in the form of a trihydrocarbylphosphite or a hexahydrocarbylphosphorous triamide, the reaction proceeds. In the absence of the catalyst, no reaction occurs.

The most preferred of this group of catalysts are trialkylphosphites and hexaalkylphosphorous triamides in which each alkyl group contains up to about 18 carbon atoms. Reactions performed in acetonitrile using triethylphosphite or hexaethylphosphorous triamide as the catalyst have been found particularly efficacious.

A wide variety of bifluoride catalysts may be used in the practice of this invention. They may be represented by the general formula:

$$Q^+HF_2^-$$

where Q is a quaternary ammonium group, a quaternary phosphonium group or a alkali metal. Illustrative quaternary ammonium bifluorides include tetramethylammonium bifluoride, tetraethylammonium bifluoride, tetrabutylammonium bifluoride, cetyltrimethylammonium bifluoride, benzyltrimethylammonium bifluoride, and the like. Typical quaternary phosphonium bifluorides which may be employed include tetramethylphosphonium bifluoride, tetrathylphosphonium bifluoride, tetrabutylphosphonium bifluoride, decyltriethylphosphonium bifluoride, and the like. The alkali metal bifluorides are lithium bifluoride, sodium bifluoride potassium bifluoride, rubidium bifluoride and cesium bifluoride.

A mixture of two or more quaternary ammonium bifluorides or of two or more quaternary phosphonium bifluorides or of two or more alkali metal bifluorides may be used as the catalyst. Likewise, mixtures of one or more quaternary ammonium bifluorides with one or more quaternary phosphonium bifluorides and/or one or more alkali metal bifluorides can be used for this purpose, if desired. Similarly one may use a mixture of one or more quaternary phosphonium bifluorides with one or more alkali metal bifluorides as the catalyst.

It is not known how or why the catalysts function in the process of this invention. Nor, is the structure or composition of the actual catalytic species known. All that is known is that when the catalyst is added to the reaction system in the form of a bifluoride of the type referred to above the reaction proceeds. Absent the catalyst, no reaction occurs.

The most preferred of this group of catalysts are potassium bifluoride and tetraalkylammonium bifluorides in which each alkyl group contains up to about 18 carbon atoms. Reactions performed in acetonitrile using such catalysts have been found particularly efficacious.

Various aminopyridine compounds may be used as catalysts in the process of this invention. These include such compounds as 2-aminopyridine, 3-aminopyridine, 4-aminopyridine and fused ring analogs thereof such as 4-aminoquinaldine, 2,3-diaminopyridine, 2,6-diaminopyridine, 3,4-diaminopyridine, and alkyl derivatives of any of the foregoing such as 2-dimethylaminopyridine, 4-di-methylaminopyridine, 4-diethylaminopyridine, and the like. Aminopyrazine and aminopyrimidines such as 2-aminopyrimidine and 4,5-diaminopyrimidine may also be used as catalyst in the process.

A mixture of two or more aminopyridines may be used as the catalyst if desired.

It is not known how or why the catalysts function in the process of this invention. Nor, is the structure or composition of the actual catalytic species known. All that is known is that when the catalyst is added to the reaction system in the form of an aminopyridine, the reaction proceeds. Absent the catalyst, no reaction occurs.

The most preferred of this group of catalysts are the monoalkyl aminopyridines and the dialkylamino pryidines in which each alkyl group contains up to about 6 carbon atoms. Reactions performed in acetonitrile using 4-dimethylaminopyridine as the catalyst have been found particularly efficacious.

When the catalyst is used together with a proton source, many different types of catalysts may be used in the process. These include alkali metal salts, trihydrocarbyl phosphites, hexahydrocarbyl phosphorous triamides, aminopyridines, quaternary ammonium bifluorides, and quaternary ammonium monofluorides.

One type of alkali metal salts that may be used are the active fluorine-containing salts, viz., potassium fluoride, rubidium fluoride, and cesium fluoride.

Another type of alkali metal salts that may be used are those which are devoid of fluorine with the proviso that they exhibit the appropriate catalytic activity. In this connection, not all such alkali metal salts exhibit a catalytic effect in the reaction and thus in any given instance where the suitability of a given fluorine-free alkali metal salt is not known, recourse should be had to the simple expedient of performing a few pilot experiments to determine whether the material will serve as a catalyst in the reaction. Alkali metal salts devoid of fluorine which are active catalysts in the process of this invention include the alkali metal azides such as lithium azide, sodium azide, and potassium azide; alkali metal cyanides such as sodium cyanide, potassium cyanide, and cesium cyanide; alkali metal hydroxides such as sodium hydroxide and potassium hydroxide; alkali metal carbonates such as potassium carbonate and cesium carbonate; and the like. Sodium carbonate, sodium nitrite, sodium phosphate are among materials deemed inactive as catalysts in the process of this invention.

Collectively, the trihydrocarbyl phosphites and the hexahydrocarbyl phosphorous triamides that may be used as catalysts in the process may be represented by the formula:

$R_3P$

wherein all of the R groups are either hydrocarbyloxy groups or dihydrocarbylamino groups. Illustrative trihydrocarbylphosphites include trimethylphosphite, triethylphosphite, tripropylphosphite, triisopropylphosphite, tributylphosphite, tridodecylphosphite, triallylphosphite, trioleylphosphite, tricyclohexylphosphite, tricyclopropylcarbinylphosphite, triphenylphosphite, tritolylphosphite, tribenzylphosphite, phenyldiethylphosphite, dibenzyloctadecylphosphite, and the like. Among the hexahydrocarbylphosphorous triamides that may be employed as catalysts are hexamethylphosphorous triamide, hexaethylphosphorous triamide, hexapropylphosphorous triamide, hexaisopropylphosphorous triamide, hexabutylphosphorous triamide, hexacyclopentylphosphorous triamide, hexaphenylphosphorous triamide, hexa(4-ethylphenyl)phosphorous triamide, hexa(2-phenethyl)phosphorous triamide, hexacrotonylphosphorous triamide, and the like.

Various aminopyridine compounds may be used as catalysts in the process of this invention. These include such compounds as 2-aminopyridine, 3-aminopyridine, 4-aminopyridine and fused ring analogs thereof such as 4-aminoquinaldine, 2,3-diaminopyridine, 2,6-diaminopyridine, 3,4-diaminopyridine, and alkyl derivatives of any of the foregoing such as 2-dimethylaminopyridine, 4-dimethylaminopyridine, 4-diethylaminopyridine, and the like. Aminopyrazine and aminopyrimidines such as 2-aminopyrimidine and 4,5-diaminopyrimidine may also be used as catalyst in the process.

Another category of catalysts for the process are the bifluorides, notably quaternary ammonium bifluorides, quaternary phosphonium bifluorides, and alkali metal bifluorides. Collectively, these bifluoride catalysts may be represented by the formula:

$Q^+HF_2^-$

wherein Q is a quaternary ammonium group, a quaternary phosphonium group, or an alkali metal. Illustrative quaternary ammonium bifluorides include tetramethylammonium bifluoride, tetraethylammonium bifluoride, tetrabutylammonium bifluoride, cetyltri- methylammonium bifluoride, benzyltrimethylammonium bifluoride, and the like. Typical quaternary phosphonium bifluorides which may be employed include tetramethylphosphonium bifluoride, tetrathylphosphonium bifluoride, tetrabutylphosphonium bifluoride, decyltriethylphosphonium bifluoride, and the like. The alkali metal bifluorides are lithium bifluoride, sodium bifluoride potassium bifluoride, rubidium bifluoride and cesium bifluoride.

Still another group of catalysts that may be employed are the quaternary ammonium monofluorides. These are exemplified by such compounds as tetramethylammonium fluoride, tetraethylammonium fluoride, tetrabutylammonium fluoride, cetyl tripropylammonium fluoride, tricaprylmethylammonium fluoride, benzyltriethylammonium fluoride, benzyltrimethylammonium fluoride, benzyltributylammonium fluoride and the like.

Still other types of catalysts for the process may now occur to those skilled in the art from a perusal of this disclosure.

Carboxylic acids, water, alcohols, polyols, phenols, and the like exemplify the proton sources that may be used in the practice of this invention. The carboxylic acids may be cyclic (e.g., benzoic acid) or non-cyclic (e.g., acetic acid) and may be monocarboxylic acids (e.g., propionic acid) or polycarboxylic acids (e.g., succinic acid). Likewise, the alcohols may be cyclic (e.g., cyclohexanol) or non-cyclic (e.g., ethanol). The polyols may be linear (e.g., ethylene glycol) or branched (e.g., pentaerythritol). The phenols may be monohydric (e.g., phenol) or polyhydric (e.g., hydroquinone) and mononuclear (e.g., cresol) or polynuclear (e.g., 4,4'-dihydroxydiphenyl).

In selecting the catalyst proton source for use in a given reaction, care should be taken to use substances which do not adversely interact with the another. For example, one should not employ a carboxylic acid promoter with alkali metal salts such as the carbonates, cyanides, or hydroxides.

The amount of catalyst used may be varied depending on the activity of the catalyst being used. Thus with some catalysts such as trihydrocarbylphosphites, hexahydrocarbylphosphorous triamides, aminopyridines, quaternary ammonium fluorides, quaternary ammonium bifluorides, and potassium carbonate, small catalytic quantities (e.g., as little as 0.10 mole per mole of quinone) may be used. With other catalysts such as KF, at least a stoichiometric amount relative to the quinone is desirable to achieve reasonable reaction rates (hours vs. days).

For best results, one should use a stoichiometric amount of the proton source relative to the quinone present in the reaction mixture. Desirably the amount of excess proton source, if used, should be kept as small as convenient, typically no more than about 5 to 10 percent above stoichiometric.

It is not known how or why the catalyst function in the process of this invention. Nor, is the structure or composition of the actual catalytic species known. All that is known is that when the catalyst is added to the reaction system in the form of an inorganic salt such as above-described, preferably in finely divided form, the reaction proceeds. When the catalyst is absent, no reaction occurs.

Of the catalyst-proton source systems described above, systems based on the above-referred to alkali metal salts, especially potassium or cesium fluorides, along with carboxylic acids, especially the lower fatty acids (acetic acid, propionic acid) are preferred.

In accordance with a particularly preferred embodiment of this invention, the reaction is performed in the presence of ammonium bifluoride which serves both as the catalyst and as the proton source. Thus with this substance it is unnecessary to use an acid, alcohol, water, or the like as a proton source.

Ordinarily the reaction will be conducted at temperatures within the range of about -20 to about 100°C, although temperatures outside this range may be found useful in particular cases. Preferably, the temperature is maintained within the range of about 0 to about 25°C throughout substantially the entire reaction period.

Quinones that may be used in the process of this invention include mononuclear and polynuclear quinones, both 1,2-quinones and 1,4-quinones. Electron donating substituents, such as hydrocarbyl groups, hydrocarbyloxy groups, amino and mono- and dihydrocarbylamino groups, the hydroxyl group, and the like may be present in the quinones. A few exemplary quinones which may be used include 1,2-benzoquinone, 1,4-benzoquinone, 2-methyl-1,4-benzoquinone, 2-methoxy-1,4-benzoquinone, 2,5-dimethoxy1,4-benzoquinone, 2-anilino-1,4-benzoquinone, 2,5-dianilino-1,4-benzoquinone, 2-phenyl-1,4-benzoquinone, polyporio acid, the ubiquinones, 2,3-dimethyl-1,4-benzoquinone, 2,5-dimethyl-1,4-benzoquinone, 1,4-naphthoquinone, 1,2-naphthoquinone, Vitamin $K_1$, Vitamin $K_2$, 2-methyl-1,4-naphthoquinone, anthraquinone, 2-methylanthraquinone, 2-ethylanthraquinone, 2-tert-butylanthraquinone, 1-aminoanthraquinone, 2-aminoanthraquinone, 1-amino-4-hydroxyanthraquinone, 1,2-diaminoanthraquinone, 1,4-diaminoanthraquinone, 1,5-diaminoanthraquinone, 2,6-diaminoanthraquinone, 1,8-diamino-4,5-dihydroxyanthraquinone, 1-hydroxy-4-(p-toluidino)anthraquinone, diphenoquinone, indanthrene blue, 1,2-dihydroxyanthraquinone, 9,10-phenanthraquinone, indanthrene violet, chrysophanic acid, and the like.

The perfluoroalkyltrihydrocarbyl silanes used in the process of this invention may be represented by the general formula

R'SiR₃

where R' is a perfluoroalkyl group, such as trifluoromethyl, pentafluoroethyl, or perfluorohexyl, and R, independently, is a hydrocarbyl group, such as alkyl, alkenyl, alkynyl, cycloalkyl, aryl, or aralkyl. The number of carbon atoms in R and R' is irrelevant so long as the silane is co-reactive with the quinone in the process. A few illustrative compounds include trifluoromethyltrimethylsilane, tridecyltrifluoromethylsilane, trifluoromethyltrivinyl-silane, triallyltrifluoromethylsilane, tricyclopentyl-trifluoromethylsilane, tricyclopropylcarbinyl-trifluoromethylsilane, trifluoromethyltriphenylsilane, trifluoromethyltri-(1-naphthyl)silane, tribenzyltrifluoromethylsilane, and corresponding and similar analogs containing the higher "homologous" perfluoroalkyl groups such as perfluoroethyl, perfluoropropyl, perfluoroisopropyl, or perfluorobutyl.

As noted above, this invention also provides gemdisubstituted cyclohexadienones in which the gem substituents are a perfluoroalkyl group and a trihydrocarbylsiloxy group. In one preferred embodiment the perfluoroalkyl group is a trifluoromethyl group. In another preferred embodiment the trihydrocarbylsiloxy group is a trialkylsiloxy group. Particularly preferred compounds are those in which the gem substituents are a trialkylsiloxy group and a trifluoromethyl group.

Among the preferred subclasses of compounds provided by this invention are the following:
4-trialkylsiloxy-4-trifluoromethyl-2,5-cyclohexadien-1-ones;
4-trialkylsiloxy-4-trifluoromethyl-2,5-cyclohexadien-1-ones having an alkyl substituent in at least the 2 or 6 position;
1,4-dihydro-1-oxo-4-trialkylsiloxy-4-trifluoromethylnaphthalenes;
9,10-dihydro-9-oxo-10-trialkylsiloxy-10-trifluoromethylanthracenes;
2-trialkylsiloxy-2-trifluoromethyl-3,5-cyclohexadien-1-ones;
2-trialkylsiloxy-2-trifluoromethyl-3,5-cyclohexadien-1-ones having an alkyl substituent in at least the 4 or 6 position; and
9,10-dihydro-9-oxo-10-trialkylsiloxy-10-trifluoromethylphenanthrenes.

Illustrative gem-disubstituted compounds of this invention include:
4-trifluoromethyl-4-trimethylsiloxy-2,5-cyclohexadien-1-one;
4-pentafluoroethyl-4-trimethylsiloxy-2,5-cyclohexadien-1-one;
4-heptafluoropropyl-4-trimethylsiloxy-2,5-cyclohexadien-1-one;
4-tricyclohexylsiloxy-4-trifluoromethyl-2,5-cyclohexadien-1-one;
4-trifluoromethyl-4-triphenylsiloxy-2,5-cyclohexadien-1-one;
4    -nonafluorobutyl-4-(4-biphenylyl)siloxy-2,5-cyclohexadien-1-one;
4-tribenzylsiloxy-4-trifluoromethyl-2,5-cyclohexadien-1-one;
2-ethyl-4-trifluoromethyl-4-tributylsiloxy-2,5-cyclohexadien-1-one;
4-trifluoromethyl-2-methoxy-4-trioctylsiloxy-2,5-cyclohexadien-1-one;
4-trifluoromethyl-2,5-dimethoxy-4-tri-(4-methylphenyl)-siloxy-2,5-cyclohexadien-1-one;
2-anilino-4-pentafluoroethyl-4-trimethylsiloxy-2,5-cyclohexadien-1-one;
2-trifluoromethyl-2-triisopropylsiloxy-3,5-cyclohexadien-1-one;
6-ethyl-2-trifluoromethyl-2-tributylsiloxy-3,5-cyclohexadien-1-one;
4,6-diethyl-2-trifluoromethyl-2-triphenylsiloxy-3,5-cyclohexadien-1-one;
1,4-dihydro-1-oxo-4-trifluoromethyl-4-trioctylsiloxynaphthalene;
1,4-dihydro-2-methyl-1-oxo-4-trifluoromethyl-4-tripropylsiloxynaphthalene;
9,10-dihydro-9-oxo-10-pentafluoroethyl-10-triethylsiloxyanthracene;
1,4-diamino-9,10-dihydro-9-oxo-10-pentafluoroethyl-10-triethylsiloxyanthracene;
9,10-dihydro-1,2-dihydroxy-9-oxo-10-pentafluoroethyl-10-triethylsiloxyanthracene;
9,10-dihydro-9-oxo-10-pentafluoroethyl-10-triethylsiloxyphenanthrene; and
9,10-dihydro-1-ethoxy-9-oxo-10-pentafluoroethyl-10-triethylsiloxyphenanthrene.

The practice and advantages of this invention will become still further apparent from the following illustrative examples. Examples I and II illustrate the preparation of perfluoroalkyltrihydrocarbylsilanes, the class of reactants used in the process of this invention. The procedure used in Examples I and II is based on Ruppert, et al. Tetrahedron Letters, 1984, 25, 2195.


EXAMPLE I


Triethyltrifluoromethylsilane


A flask equipped with a dry ice condenser was flame dried under a nitrogen stream, and charged with 25g (0.17 mol) of chlorotriethylsilane and 40 mL of dichloromethane. After cooling the resulting solution to -78°C and charging the condenser with dry ice and acetone, 40 mL (0.43 mol) of bromotrifluoromethane (Freon 13B1) that had been condensed into a graduated tube was warmed to room temperature and allowed to distill into the flask. The cold solution was treated dropwise with 66mL (0.24 mol) of hexaethylphosphorous triamide, allowed to stir at -78°C for two hours, and allowed to stir at room temperature overnight. Low boiling components were then short path distilled into a cold (-78°C) receiving flask at > 1 torr with the pot temperature kept at < 50°C. The distillate was further fractionated by removal of the dichloromethane (40-45°C at atmospheric pressure) and short path distillation to give 22.0 g of 98% pure (69% yield) triethyltrifluoromethylsilane: bp 52-54°C at 10 torr; $^1$H NMR (CDCl$_3$) δ 0.59-1.16 (m); $^{19}$F NMR (CDCl$_3$, relative to CFCl$_3$) -61.3 ppm (s); IR (neat) 2960, 2915, 2882, 1458, 1413, 1206, 1055, 1020, 734, 693 cm$^{-1}$; mass spectrum (70 eV) m/z (relative intensity) 115 (66, M-CF$_3$), 105 (46), 87 (85), 77 (100), 59 (56), 49 (41), 47 (37), 41 (38). Anal. Calcd. for C$_7$H$_{15}$F$_3$Si: C, 45.62; H, 8.20. Found: C, 47.53; H, 8.56.

## EXAMPLE II

### Tri-n-butyltrifluoromethylsilane

A flask equipped with a dry ice condenser was flame dried under a nitrogen stream, and charged with 5.0g (20 mmol) of chlorotri-n-butylsilane and 10 mL of dichloromethane. After cooling the resulting solution to -78°C and charging the condenser with dry ice and acetone, 6.2 mL (66 mmol) of bromotrifluoromethane (Freon 13B1) that had been condensed into a graduated tube was warmed to room temperature and allowed to distill into the flask. The cooling bath was removed and the mixture was allowed to warm to the temperature of the refluxing Freon (-59°C). To this cold solution was added, dropwise, 8.0 mL (29 mmol) of hexaethylphosphorous triamide. The resulting solution was stirred at reflux for 1 hour. Removal of the condenser and continued stirring for 1 hour resulted in evaporation of excess Freon and warming of the solution to room temperature. Dilution with 30 mL of dichloromethane, water (three 30 mL portions) and 1N HCl (two 30 mL portions) washing, drying (MgSO$_4$), and concentration afforded a residue which was short path distilled to give 3.6g (64% yield) of tri-n-butyltrifluoromethylsilane: bp 53-58°C at 0.5 torr; $^1$H NMR (CDCl$_3$) δ 0.60-1.10 (m, 5H), 1.10-1.56 (m, 4H); $^{19}$F NMR (CDCl$_3$, relative to CFCl$_3$) -61.6 ppm (s); IR (neat) 2956, 2925, 2872, 1214, 1058 cm$^{-1}$; mass spectrum (70 eV) m/z (relative intensity) 199 (30, M-CF$_3$), 143 (80), 105 (30), 101 (27), 87 (30), 77 (66), 63 (43), 59 (41), 55 (54), 47 (25), 43 (20), 41 (100). Anal. Calcd. for C$_{13}$H$_{27}$F$_3$Si: C, 58.16; H, 10.14. Found: C, 58.26; H, 10.09.

Examples III through XXIII illustrate the gem-disubstituted compounds of this invention and methods by which they may be prepared.

## EXAMPLE III

### 4-Triethylsiloxy-4-trifluoromethyl-2,5-cyclohexadien-1-one

A mixture of 165 mg (1.5 mmol) of 1,4-benzoquinone, 332 mg (18 mmol) of triethyltrifluoromethylsilane, 15 mg (0.31 mmol) of sodium cyanide, and 2 mL of acetonitrile was stirred vigorously at 25°C for 42 hours. The mixture was filtered and the filtrate was concentrated in vacuo. Purification of the resulting residue by preparative thin layer chromatography (one 2 mm silica gel plate eluted with 50% dichloromethane - 50% petroleum ether) afforded 178 mg (41% yield) of 4-triethylsiloxy-4-trifluoromethyl-2,5-cyclohexadien-1-one as a pale yellow liquid: bp 68-78°C at 0.5 torr; $^1$H NMR (CDCl$_3$) δ 0.40-1.06 (m, 15H), 6.41 (d, 2H, J = 9Hz), 6.89 (d, 2H, J = 9Hz); $^{19}$F NMR (CDCl$_3$, relative to CFCl$_3$) -83.8 ppm (t, J$_{FH}$ = 4 Hz); IR (neat) 2956, 2912, 2877, 1677, 1611, 12.65, 12.40, 1182, 1129, 1067, 1004, 835, 749, 732 cm$^{-1}$; mass spectrum (70 eV) m/z (relative intensity) 263 (6, M-C$_2$H$_5$), 139 (79), 111 (100) 105 (68), 83 (41), 77 (100), 47 (31), 45 (35). Anal. Calcd. for C$_{13}$H$_{19}$F$_3$O$_2$Si: C, 53.39; H, 6.54. Found: C, 53.60; H, 6.79.

## EXAMPLE IV

### 4-Triethylsiloxy-4-trifluoromethyl-2,5-cyclohexadien-1-one

The procedure of Example III was repeated except that the catalyst was 293 mg (4.5 mmol) of potassium cyanide and the reaction period was but one hour. The 4-triethylsiloxy-4-trifluoromethyl-2,5-cyclohexadien-1-one was recovered in 64% yield.

## EXAMPLE V

### 4-Triethylsiloxy-4-trifluoromethyl-2,5-cyclohexadien-1-one

A mixture of 83 mg (0.77 mmol) of 1,4-benzoquinone, 1.66 mg (0.90 mmol) of triethyltrifluoromethylsilane, 92 mg (2.3 mmol) of powdered sodium hydroxide, and 1 mL of acetonitrile was stirred at 25°C for 2 hours. The reaction mixture was poured into 20 mL of water and the resulting aqueous mixture was extracted with two 5 mL portions of dichloromethane. Combination, drying ($MgSO_4$), and concentration of the organic layers afforded a residue which was purified by PTLC, giving 66 mg (29% yield) of 4-triethylsiloxy-4-trifluoromethyl-2,5-cyclohexadien-1-one.

## EXAMPLE VI

### 4-Triethylsiloxy-4-trifluoromethyl-2,5-cyclohexadien-1-one

A mixture of 83 mg (0.77 mmol) of 1,4-benzoquinone, 166 mg (0.90 mmol) of triethyltrifluoromethylsilane, 113 mg (2.3 mmol) of ground lithium azide, and 1 mL of acetonitrile was stirred at 25°C for 22 hours. The reaction mixture was poured into 20 mL of 1N HCl and the resulting aqueous mixture was extracted with three 10 mL portions of dichloromethane. Combination, drying ($MgSO_4$), and concentration of the organic layers afforded a residue which was purified by PTLC, giving 96 mg (43% yield) of 4-triethylsiloxy-4-trifluoromethyl-2,5-cyclohexadien-1-one.

## EXAMPLE VII

### 4-Triethylsiloxy-4-trifluoromethyl-2,5-cyclohexadien-1-one

The procedure of Example III was repeated using a mixture of 86 mg (0.80 mmol) of 1,4-benzoquinone, 147 mg (0.80 mmol) of triethyltrifluoromethylsilane, 22 mg (0.16 mmol) of potassium carbonate, and 1 mL of acetonitrile at 25°C for 7 hours. PTLC afforded 120 mg (51% yield) of 4-triethylsiloxy-4-trifluoromethyl-2,5-cyclohexadien-1-one.

## EXAMPLE VIII

### 4-Triethylsiloxy-4-trifluoromethyl-2,5-cyclohexadien-1-one

The procedure of Example VI was repeated using a mixture of 86 mg (0.80 mmol) of 1,4-benzoquinone, 147 mg (0.80 mmol) of triethyltrifluoromethylsilane, 22 mg (0.16 mmol) of potassium carbonate, and 1 mL of N,N-dimethylformamide at 25°C for 1 hour. Extraction of the aqueous mixture with diethylether and PTLC afforded 123 mg (53% yield) of 4-triethylsiloxy-4-trifluoromethyl-2,5-cyclohexadien-1-one was achieved.

## EXAMPLE IX

### 2,6-Di-tert-butyl-4-Triethylsiloxy-4-trifluoromethyl-2,5-cyclohexadien-1-one

The procedure of Example III was repeated using a mixture of 176 mg (0.80 mmol) of 2,6-di-tert-butyl-1,4-benzoquinone, 147 mg (0.80 mmol) of triethyltrifluoromethylsilane, 22 mg (0.16 mmol) of potassium carbonate, and 1 mL of N,N-dimethylformamide at 25°C for 21.5 hours. PTLC afforded 133 mg (41% yield) of 2,6-di-tert-butyl-4-triethylsiloxy-4-trifluoromethyl-2,5-cyclohexadien-1-one as a colorless liquid: $^1H$ NMR ($CDCl_3$) $\delta$ 0.39-1.10 (m, 15H), 1.22 (s, 18H), 650 (s, 2H); $^{19}F$ NMR ($CDCl_3$, relative to $CFCl_3$) -80.5 ppm (s); IR (neat) 2957, 2910, 2876, 1670, 1648, 1457, 1364, 1333, 1272, 1255, 1178, 1151, 1070, 1033, 994, 880, 845, 747 cm$^{-1}$; mass spectrum (70 eV) m/z (relative intensity) 375 (60, M-$C_2H_5$), 335 (50), 299 (20), 57 (100) 41 (40). Anal. Calcd. for $C_{21}H_{35}F_3O_2Si$: C, 62.34; H, 8.72. Found: C, 63.05; H, 8.75.

## EXAMPLE X

### 4,6-Di-tert-butyl-2-Triethylsiloxy-2-trifluoromethyl-3,5-cyclohexadien-1-one

The procedure of Example III was repeated using a mixture of 176 mg (0.80 mmol) of 3,5-di-tert-butyl-1,2-benzoquinone, 147 mg (0.80 mmol) of triethyltrifluoromethylsilane, 22 mg (0.16 mmol) of potassium carbonate, and 1 mL of acetonitrile at 25°C for 24 hours. PTLC afforded 269 mg (83% yield) of 4,6-di-tert-butyl-2-triethylsiloxy-2-trifluoromethyl-3,5-cyclohexadien-1-one as a yellow liquid: $^1H$ NMR ($CDCl_3$) $\delta$ 0.35-1.10 (m, 15H), 1.16 (s, 3H), 1.23 (s, 3H), 5.85 (d, 1H, J = 2 Hz), 6.84 (d, 1H, J = 2 Hz); $^{19}F$ NMR ($CDCl_3$, relative to

CFCl₃) -80.0 ppm (s); IR (neat) 2955, 2909, 2874, 1689, 1459, 1366, 1270, 1244, 1179, 1143, 1072, 1033, 998, 890, 831, 810, 743, 695 cm$^{-1}$; mass spectrum (70 eV) m/z (relative intensity) 404 (29, M$^+$), 389 (20), 375 (79, M-C$_2$H$_5$), 57 (100). Anal. Calcd. for C$_{21}$H$_{35}$F$_3$O$_2$Si: C, 62.34; H, 8.72. Found: C, 62.46; H, 8.69.

## EXAMPLE XI

### 1,4-Dihydro-1-oxo-4-triethylsiloxy-4-trifluoromethylnaphthalene

The procedure of Example VIII was repeated using a mixture of 127 mg (0.80 mmol) of 1,4-naphthoquinone, 147 mg (0.80 mmol) of triethyltrifluoromethylsilane, 22 mg (0.16 mmol) of potassium carbonate, and 1 mL of N,N-di-methylformamide at 25°C for 2 hours. PTLC afforded 113 mg (41% yield) of 1,4-dihydro-1-oxo-4-triethylsiloxy-4-trifluoromethylnaphthalene as a brown liquid: ¹H NMR (CDCl₃) δ 0.30-1.00 (m, 15H), 6.65 (d, 1H, J = 10 Hz), 7.06 (d, 1H, J = 10 Hz), 7.50-8.30 (m, 4H); ¹⁹F NMR (CDCl₃, relative to CFCl₃) -79.6 ppm (s); IR (neat) 22956, 2911, 2876, 1676, 1599, 1454, 1379, 1299, 1253, 178, 1154, 1110, 1065, 1058, 1018, 952, 836, 767, 748, 732 cm$^{-1}$; mass spectrum (70 eV) m/z (relative intensity) 313 (40, M-C$_2$H$_5$), 189 (55), 161 (100), 133 (55), 77 (30). Anal. Calcd. for C$_{17}$H$_{21}$F$_3$O$_2$Si: C, 59.62; H, 6.18. Found: C, 59.91; H, 6.33.

## EXAMPLE XII

### 9,10-Dihydro-9-oxo-10-triethylsiloxy-10-trifluoromethylanthracene

The procedure of Example VIII was repeated using a mixture of 167 mg (0.80 mmol) of 9,10-anthraquinone, 147 mg (0.80 mmol) of triethyltrifluoromethylsilane, 22 mg (0.16 mmol) of potassium carbonate, and 2 mL of N,N-di-methylformamide at 25°C for 5 hours. PTLC afforded 258 mg (82% yield) of 9,10-dihydro-9-oxo-10-triethylsiloxy-10-trifluoromethylanthracene as a colorless liquid: ¹H NMR (CDCl₃) δ 0.19-0.90 (m, 15H), 7.50 - 7.90 (m, 4H), 7.90-8.13 (m, 2H), 8.30-8.47 (m, 2H); ¹⁹F NMR (CDCl₃, relative to CFCl₃) -79.9 ppm (s) (t, J$_{FH}$ = 14 Hz); IR (neat) 2959, 2931, 2875, 1667, 1589, 1455, 1412, 1320, 1272, 1245, 1179, 1134, 1087, 1012, 950, 932, 893, 841, 817, 763, 736, 715, 692, 662, 630, 596 cm$^{-1}$; mass spectrum (70 eV) m/z (relative intensity) 363 (10, M-C$_2$H$_5$), 211 (100), 183 (25), 77 (35). Anal. Calcd. for C$_{21}$H$_{23}$F$_3$O$_2$Si: C, 64.26; H, 5.91. Found: C, 64.26; H, 6.01.

## EXAMPLE XIII

### 9,10-Dihydro-9-oxo-10-triethylsiloxy-10-trifluoromethylphenanthrene

The procedure of Example VIII was repeated using a mixture of 167 mg (0.80 mmol) of 9,10-phenanthrenequinone, 147 mg (0.80 mmol) of triethyltrifluoromethylsilane, 22 mg (0.16 mmol) of potassium carbonate, and 2 mL of N,N-dimethylformamide at 25°C for 4 hours. PTLC afforded 260 mg (83% yield) of 9,10-dihydro-9-oxo-10-tri-ethylsiloxy-10-trifluoromethylphenanthrene as a pale yellow liquid: ¹H NMR (CDCl₃) δ 0.45-1.17 (m, 15H), 7.31-8.10 (m, 8H); ¹⁹F NMR (CDCl₃, relative to CFCl₃) -79.4 ppm (s); IR (neat) 2953, 2909, 2874, 1707, 1598, 1450, 1298, 1279, 1251, 1230, 1178, 1117, 1029, 1009, 944, 918, 837, 778, 766, 757, 731, 630 cm$^{-1}$; mass spectrum (70 eV) m/z (relative intensity) 363 (49, M-C$_2$H$_5$), 294 (100), 236 (29). Anal. Calcd. for C$_{21}$H$_{23}$F$_3$O$_2$Si: C, 64.26; H, 5.91. Found: C, 64.28; H, 5.92.

## EXAMPLE XIV

### 4-Tributylsiloxy-4-trifluoromethyl-2,5-cyclohexadien-1-one

A mixture of 86 mg (0.80 mmol) of 1,4-benzoquinone, 215 mg (0.80 mmol) of tri-n-butyltrifluoromethylsilane, 22 mg (0.16 mmol) of ground potassium carbonate, and 1 mL of N,N-dimethylformamide was stirred vigorously at room temperature for one hour and poured into 10 mL of 1N HCl. The resulting aqueous mixture was extraced with three 10 mL portions of diethyl ether. Combination, drying (MgSO₄), and concentration of the ether layers afforded a residue which was purified by PTLC (one 2-mm silica gel plate eluted with 25% dichloromethane - 75% petroleum ether) to give 156 mg (52% yield) of 4-tri-n-butylsiloxy-4-trifluoromethyl-2,5-cyclohexadien-1-one: ¹H NMR (CDCl₃) δ 0.50-0.61 (m, 6H); 0.85 (t, 9H, J = 5 Hz), 1.19-1.35 (m, 12H), 6.39 (d, 2H, J = 9 Hz), 6.82 (d, 2H, J = 9 Hz); $^{19}$F NMR (CDCl$_3$, relative to CFCl$_3$) -80.0 ppm (s); IR (KBr) 2960, 2920, 2870, 2865, 1692, 1680, 1640, 1380, 1265, 1240, 1180, 1130, 1080, 1065, 1005, 990, 835, cm$^{-1}$; mass spectrum (70 eV) m/z (relative intensity) 319 (5, M-C$_4$H$_9$), 307 (48), 161 (20), 139 (39), 121 (52), 111 (75), 105 (62), 93 (72), 83 (29), 77 (100), 65 (24), 63 (50), 61 (24), 55 (68), 44 (31), 43 (35), 41 (35). Anal. Calcd. for C$_{19}$H$_3$·F$_3$O$_2$Si: C, 60.60; H, 8.30. Found: C, 60.76; H, 8.43.

## EXAMPLE XV

### 4-Triethylsiloxy-4-trifluoromethyl-2,5-cyclohexadien-1-one

A mixture of 83 mg (0.77 mmol) of 1,4-benzoquinone, 1.66 mg (0.90 mmol) of triethyltrifluoromethylsilane, and 1 mL of acetonitrile was treated with one drop of hexaethylphosphorous triamide, stirred at 25° C for 23.5 hours, and poured into 20 mL of water. The resulting aqueous mixture was extracted with three 10 mL portions of dichloromethane. Combination, drying (MgSO$_4$), and concentration of the organic layers afforded a residue which was purified by preparative thin layer chromatography (one 2 mm silica gel plate developed with 50% dichloromethane - 50% petroleum ether), giving 142 mg (63% yield) of 4-triethylsiloxy-4-trifluoromethyl-2,5-cyclohexadien-1-one as an amber liquid: $^1$H NMR (CDCl$_3$) δ 0.40-1.06 (m, 15 H), 6.41 (d, 2H, J = 9 Hz), 6.89 (d, 2H, J = 9 Hz); $^{19}$F NMR (CDCl$_3$, relative to CFCl$_3$) -83.8 ppm (t, J$_{FH}$ = 4 Hz); IR (neat) 2956, 2912, 2877, 1677, 1611, 1265, 1240, 1182, 1129, 1067, 1004, 835, 749, 732 cm$^{-1}$; mass spectrum (70 eV) m/z (relative intensity) 263 (6, M-C$_2$H$_5$), 139 (79), 111 (100), 105 (68), 83 (41), 77 (100), 47 (31), 45 (35). Anal. Calcd. for C$_{13}$H$_{19}$F$_3$O$_2$Si: C, 53.39; H, 6.54. Found: C, 53.60; H, 6.79.

## EXAMPLE XVI

### 4-Triethylsiloxy-4-trifluorometyl-2,5-cyclohexadien-1-one

The procedure of Example XV was repeated except that 26 microliters (0.15 mmol) of triethylphosphite was used in place of hexaethylphosphorous triamide, the reaction time was 21 hours, and the reaction mixture was simply concentrated in vacuo to give a residue which was purified by PTLC to give 115 mg (51% yield) of 4-triethylsiloxy-4-trifluoromethyl-2,5-cyclohexadien-1-one.

## EXAMPLE XVII

### 4-Triethylsiloxy-4-trifluoromethyl-2,5-cyclohexadien-1-one

A mixture of 83 mg (0.77 mmol) of 1,4-benzoquinone, 166 mg (0.9 mmol) of triethyltrifluoromethylsilane, and 1 mL of acetonitrile was treated with 22mg (0.077 mmol) of tetrabutylammonium bifluoride and stirred at 25° C for 30 minutes. Concentration of the mixture afforded a black oil which was purified by means of preparative thin layer chromatography (one 2 mm silica gel plate eluted with 50% dichloromethane-50% petroleum ether) to give 74 mg (33% yield) of 4-triethylsiloxy-4-trifluoromethyl-2,5-cyclohexadien-1-one.

## EXAMPLE XVIII

### 4-Triethylsiloxy-4-trifluoromethyl-2,5-cyclohexadien-1-one

A mixture of 100 mg (1.3 mmol) of potassium bifluoride, 119 mg (1.1 mmol) of 1,4-benzoquinone, and 2 mL of acetonitrile was treated with 239 mg (1.3 mmol) of triethyltrifluoromethylsilane and stirred vigorously at room temperature for 2 hours. The mixture was filtered and the filter cake was washed with dichloromethane. Concentration of the combined filtrates gave a black oil which was dissolved in dichloromethane and loaded onto a column of silica gel. The column was washed with dichloromethane until the eluent contained no uV active material. Concentration of the eluent gave a colorless oil which was purified by PTLC (one 2 mm silica gel plate eluted with 50% dichloromethane-50% petroleum ether) to give 134 mg (41% yield) of 4-triethylsiloxy-4-trifluoromethyl-2,5-cyclohexadien-1-one.

## EXAMPLE XIX

## 4-Triethylsiloxy-4-trifluoromethyl-2,5-cyclohexadien-1-one

A mixture of 86 mg (0.80 mmol) of 1,4-benzoquinone, 147 mg (0.80 mmol) of triethyltrifluoromethylsilane, 5 mg (0.04 mmol) of 4-dimethylaminopyridine, and 1 mL of acetonitrile was stirred at 25°C overnight and concentrated in vacuo. The resulting residue was purified by preparative thin layer chromatography (one 2 mm silica gel plate eluted with 50% dichloromethane-50% petroleum ether), to give 116 mg (50% yield) of 4-triethylsiloxy-4-trifluoromethyl-2,5-cyclohexadien-1-one as an amber liquid: $^1$H NMR (CDCl$_3$) $\delta$ 0.40-1.06 (m, 15 H), 6.41 (d, 2H, J = 9 Hz), 6.89 (d, 2H, J = 9 Hz); $^{19}$F NMR (CDCl$_3$, relative to CFCl$_3$) -83.8 ppm (t, $J_{FH}$ = 4 Hz); IR (neat) 2956, 2912, 2877, 1677, 1611, 1265, 1240, 1182, 1129, 1067, 1004, 835, 749, 732 cm$^{-1}$; mass spectrum (70 eV) m/z (relative intensity) 263 (6, M-C$_2$H$_5$), 139 (79), 111 (100), 105 (68), 83 (41), 77 (100), 47 (31), 45 (35). Anal. Calcd. for C$_{13}$H$_{19}$F$_3$O$_2$Si: C, 53.39; H, 6.54. Found: C, 53.60; H, 6.79.

## EXAMPLE XX

### 4-Triethylsiloxy-4-trifluoromethyl-2,5-cyclohexadien-1-one

The procedure of Example XIX was repeated with the exception that the temperature was maintained at 0-5°C and the reaction time was 3 hours. The yield of 4-triethylsiloxy-4-trifluoromethyl-2,5-cyclohexadien-1-one was 55%.

## EXAMPLE XXI

### 4-Hydroxy-4-trifluoromethyl-2,5-cyclohexadien-1-one

A mixture of 74 mg (1.3 mmol) of ground ammonium bifluoride (NH$_4$HF$_2$), 119 mg (1.1 mmol) of 1,4-benzoquinone, and 2 mL of acetonitrile was treated with 239 mg (1.3 mmol) of triethyltrifluoromethylsilane and stirred vigorously at room temperature for 4 hours. The mixture was filtered and the filter cake was washed with dichloromethane. Concentration of the combined filtrates gave a black solid which was triturated with dichloromethane. The resulting mixture was filtered and the filter cake was washed with dichloromethane. Concentration of the combined filtrates afforded a brown solid which was purified by PTLC (one 2 mm silica gel plate eluted with a 2% methanol-98% dichloromethane) to give 83 mg (42% yield) of 4-hydroxy-4-tri-fluoromethyl-2,5-cyclohexadien-1-one.

## EXAMPLE XXII

### 4.6-Di-tert-butyl-2-hydroxy-2-trifluoromethyl-3,5-cyclohexadiene-1-one

A mixture of 100 mg (1.7 mmol) of potassium fluoride, 132 mg (0.60 mmol) of 3,5-di-tert-butyl-1,2-benzoquinone, and 2 mL of acetonitrile was treated successively with 40 microliters (0.70 mmol) of glacial acetic acid and 129 mg (0.70 mmol) of triethyltrifluoromethylsilane, and stirred vigorously at room temperature for 15 minutes. The mixture was filtered and the filter cake was washed with dichloromethane. Concentration of the combined filtrates afforded a residue which was purified by PTLC (one 2 mm silica gel plate eluted with 20% dichloromethane - 80% petroleum ether) to give 27 mg (16% yield) of 4,6-di-tert-butyl-2-hydroxy-2-trifluoromethyl-3,5-cyclohexadiene-one.

## EXAMPLE XXIII

### 1.4-Dihydro-4-hydroxy-1-oxo-4-trifluoromethylnaphthalene

A mixture of 74 mg (1.3 mmol) of ground ammonium bifluoride, 174 mg (1.1 mmol) of 1,4-naphthoquinone, and 1 mL of N,N-dimethylformamide was treated with 239 mg (1.3 mmol) of triethyltrifluoromethylsilane and stirred vigorously at room temperature for one hour. Gas chromatographic analysis of the reaction mixture showed that the major naphthalene-desired product was 1,4-dihydro-4-hydroxy-1-oxo-4-trifluoromethylnaphthalene.

Comparative Examples A and B presented below indicate that two of the fluorine containing catalysts recommended by Wang as effective in the reactions described in U. S. 4,634,787 are ineffective in the reactions of this invention.

## COMPARATIVE EXAMPLE A

### Attempted Use of Sodium Fluoride as Catalyst

A mixture of 83 mg (0.77 mmol) of 1,4-benzoquinone, 166 mg (0.90 mmol) of triethyltrifluoromethylsilane, and 1 mL of acetonitrile was treated with 97 mg (2.3 mmol) of sodium fluoride (dried at 180°C, 25 torr overnight) and stirred vigorously at room temperature for 3 days. A gas chromatographic analysis showed that no reaction occurred.

## COMPARATIVE EXAMPLE B

### Attempted Use of Calcium Fluoride as Catalyst

A mixture of 46 mg (0.4 mmol) of benzoquinone, 92 mg (0.5 mmol) of triethyltrifluoromethylsilane, 102 mg (1.3 mmol) of calcium fluoride, and 1 mL of acetonitrile was stirred at room temperature for 1 hour. A gas chromatographic analysis showed no reaction occurred.

The novel gem-disubstituted cyclohexadienones of this invention are eminently useful in the synthesis of a wide variety of perfluroalkyl substituted aromatic compounds, many of which are themselves novel and of considerable utility. For example, the gem-disubstituted cyclohexadienones can be reduced using suitable metal reductant systems to perfluoroalkylated phenols. Likewise, the cyclohexadienones of this invention can be subjected to reductive amination to produce perfluoroalkylated aromatic amines. Procedures useful in effecting such reductions and reductive aminations are illustrated in Examples XXIV through XXIX below.

## EXAMPLE XXIV

### 4-Trifluoromethylphenol

A solution of 300 mg (1.0 mmol) of 4-triethylsiloxy-4-trifluomethyl-2,5-cyclohexadien-1-one in 1 mL of absolute ethanol was treated successively with 134 mg (2.0 mmol) of zinc dust and 1 mL of a solution of 80% acetic acid - 20% water.

The mixture was heated to reflux in a 120 5°C oil bath for one hour, allowed to cool to room temperature, and poured into 10 mL of water. The resulting aqueous mixture was extracted with three 10 mL portions of diethyl ether. Combination, drying (MgSO$_4$), and concentration of the ether layers afforded a residue which was subjected to PTLC (one 2 mm plate eluted with 20% petroleum ether - 80% dichloromethane). Removal of the UV-active band from the plate afforded a mixture of triethylsilanol (23 area percent by gas chromatography) and 4-trifluoromethylphenol (71 area percent by gas chromatography): mass spectrum (70 eV) m/z (relative intensity) 162 (100, M$^+$), 143 (56), 112 (31), 39 (22).

## EXAMPLE XXV

### 2,6-Di-tert-butyl-4-trifluoromethylphenol

A strip of aluminum foil weighing 264 mg (9.8 mmol) was amalgamated by immersion in a solution of 2% mercuric chloride in water for 15 seconds, washed with absolute ethanol followed by diethyl ether, cut into small pieces, and added to a solution of 412 mg of 96% pure (0.98 mmol) 2,6-di-tert-butyl-4-triethylsiloxy-4-trifluoromethyl-2,5-cyclohexadien-1-one in 25 mL of 10% water - 90% tetrahydrofuran. The resulting mixture was heated at 70°C for 1.5 hours, allowed to cool to room temperature, and filtered. The filter cake was washed with tetrahydrofuran. Concentration of the combined filtrates gave a residue which was poured into 25 mL of water. The aqueous mixture was extracted with three 10 mL portions of dichloromethane. Combination drying (MgSO$_4$), and concentration of the organic layers gave a residue which was purified by PTLC (one 2 mm silica gel plate eluted with petroleum ether), affording 247 mg of 95% pure (87% yield) 1,6-di-tert-butyl-4-trifluoromethylphenol. An analytical sample was obtained by crystallization from methanol: mp 78-80°C; $^1$H NMR (CDCl$_3$) δ 1.45 (s, 18H), 5.56 (broad s, 1H), 7.50 (s, 1H); $^{19}$F NMR (CDCl$_3$, relative to CFCl$_3$) -61.7 ppm (s); IR (KBr) 3632, 2963, 1337, 1319, 1241, 1167, 1141, 1109, 893, 668 cm$^{-1}$; mass spectrum (70 eV) m/z (relative intensity) 274 (20, M$^+$), 259 (100), 231 (28), 57 (57), 41 (54). Anal. Calcd. for C$_{15}$H$_{21}$F$_3$O: C, 65.67; H, 7.72. Found: C, 65.46; H, 7.94.

## EXAMPLE XXVI

## 2,4-Di-tert-butyl-4-trifluoromethylphenol

A strip of aluminum foil weighing 267 mg (9.9 mmol) was amalgamated by immersion in a solution of 2% mercuric chloride in water for 15 seconds, washed with absolute ethanol followed by diethyl ether, cut into small pieces, and added to a solution of 400 mg (0.99 mmol) of 4,6-di-tert-butyl-2-triethylsiloxy-2-trifluoromethyl-3,5-cyclohexa dien-1-one in 25 mL of 10% water - 90% tetrahydrofuran. The resulting mixture was heated at $70°$ C for 1.5 hours, allowed to cool to room temperature, and filtered. The filter cake was washed with 10 mL of tetrahydrofuran. Concentration of the combined filtrates gave a residue which was poured into 25 mL of water. The aqueous mixture was extracted with three 10 mL portions of dichloromethane. Combination, drying and concentration of the organic layers gave a residue which was purified by PTLC (one 2 mm silica gel plate eluted with petroleum ether), affording 226 mg (83% yield) of 2,4-di-tert-butyl-6-trifluoromethylphenol as a colorless liquid: $^1$H NMR (CDCl$_3$) $\delta$ 1.31 (s, 9H), 1.45 (s, 9H), 5.56 (q, H, $J_{HF}$ = 4Hz), 7.39 (d, 1H, J = 2Hz), 7.54 (d, 1H, J = 2Hz); IR (neat) 3624, 2959, 2906, 2868, 1481, 1458, 1448, 1363, 1340, 1263, 1251, 1170, 1126, 1097, 887, 694 cm$^{-1}$; mass spectrum (70 eV) m/z (relative intensity) 274 (20, M$^+$), 259 (100), 239 (68), 98 (20), 57 (22), 41 (34).

## EXAMPLE XXVII

## 4-Trifluoromethylphenol

A solution of 3.9 g (20 mmol) of 4-tri-n-butylsiloxy-4-trifluoromethyl-2,5-cyclohexadien-1-one in 10 mL of absolute ethanol was treated successively with 1.3 g (20 mmol) of zinc dust and 10 mL of a solution of 80% acetic acid - 20% water. The mixture was heated to reflux for one hour, allowed to cool to room temperature, and poured into 100 mL of water. The resulting aqueous mixture was extracted with three 50 mL portions of diethyl ether. Combination, drying (MgSO$_4$), and concentration of the ether layers afforded a residue which purified by short path distillation at 5.0 torr. At $60-65°$ C, 0.80 g (47% yield) of 4-trifluoromethylphenol was collected.

## EXAMPLE XXVIII

## 4-Trifluoromethyl-1-naphthol

A strip of aluminum foil weighing 278 mg (10 mmol) was amalgamated by immersion in a solution of 2% mercuric chloride in water for 15 seconds, washed with absolute ethanol followed by diethyl ether, cut into small pieces, and added to a solution of 353 mg (1.0 mmol) of 1,4-di-hydro-1-oxo-4-triethylsiloxy-4-trifluoromethylnaphthalene in 10 mL of 10% water - 90% tetrahydrofuran. The resulting mixture was heated at $70°$ C for 1.5 hours, allowed to cool to room temperature, and filtered. The filter cake was washed with diethyl ether. Concentration of the combined filtrates gave a residue which was poured into 25 mL of water. The aqueous mixture was extracted with three 10 mL portions of dichloromethane. Combination, drying (MgSO$_4$), and concentration of the organic layers gave a residue which was purified by PTLC (one 2 mm silica gel plate eluted with dichloromethane), affording 190 mg (90% yield) of 4-trifluoromethyl-1-naphthol as a white solid. An analytical sample was obtained by recrystallization from dichloromethane-hexane: mp $132-133°$ C; $^1$H NMR (CDCl$_3$) $\delta$ 5.50 (broad s, 1H), 6.79 (d, 1H, J = 8Hz), 7.50-7.80 (m, 3H), 8.10 - 8.45 (m, 2H); $^{19}$F NMR (CDCl$_3$, relative to CFCl$_3$) -59.5 ppm (s); IR (KBr) 3327, 1580, 1385, 1355, 1327, 1260, 1251, 1241, 1195, 1178, 1146, 1120, 1111, 1101, 1056, 767, cm$^{-1}$; mass spectrum (70 eV) m/z (relative intensity) 212 (100, M$^+$), 133 (32), 115 (100). Anal. Calcd. for C$_{11}$H$_7$F$_3$O: C, 62.27; H, 3.33. Found: C, 61.82; H, 3.50.

## EXAMPLE XXIX

## 4-Trifluoromethylaniline

A mixture of 400 mg (1.4 mmol) of 4-triethylsiloxy4-trifluoromethyl-2,5-cyclohexadien-1-one, 572 mg (4.2 mmol) of ethyl glycinate hydrochloride, 298 mg (3.6 mmol) of sodium bicarbonate, and 10 mL of 95% ethanol was heated to reflux for 6 hours, allowed to cool to room temperature, and poured into 25 mL of water. The resulting aqueous mixture was extracted with three 10 mL portions of dichlormethane. The organic layers were combined and extracted with six 5 mL portions of 1N HCl. Combination of the aqueous layers and treatment with solid sodium bicarbonate until neutral to pH paper gave a cloudy mixture that was extracted with three 10 mL portions of dichloromethane. The organic layers were combined, dried (MgSO$_4$), and stripped to give a residue which was purified by PTLC (one 2 mm silica gel plate eluted with dichloromethane), affording 160 mg (73% yield) of 4-trifluoromethylaniline.

Ortho-hydrocarbyl perfluoroalkyl phenolic compounds such as 2-alkyl- and 2,6-dialkyl-4-perfluoroalkylphenols, 2-alkyl-4-perfluoroalkylnaphthols and 6-alkyl- and 4,6-alkyl-2-perfluoroalkylphenols may be used as antioxidants and stablizers in polymers, lubricants and like substrates normally susceptible to oxidative deterioration during storage or use, and as intermediates for the synthesis of phosphites, thiophosphites, phosphates, thiophosphates, and like products which may be used as antioxidants and as agricultural chemicals. Exemplary ortho-hydrocarbyl perfluoroalkyl phenolic compounds of this type include:
2-methyl-4-perfluoromethylphenol
2-ethyl-4-perfluoromethylphenol
2-isopropyl-4-perfluoromethylphenol
2-tert-butyl-4-perfluoromethylphenol
2-(2-octyl)-4-perfluoromethylphenol
2-benzyl-4-perfluoromethylphenol
2-cyclopentyl-4-perfluoromethylphenol
2,6-dimethyl-4-perfluoromethylphenol
2,6-diethyl-4-perfluoromethylphenol
2,6-diisopropyl-4-perfluoromethylphenol
2,6-di-tert-butyl-4-perfluoromethylphenol
2-tert-butyl-6-methyl-4-perfluoromethylphenol
2-benzyl-6-methyl-4-perfluoromethylphenol
2-cyclopentyl-6-ethyl-4-perfluoromethylphenol
2-ethyl-4-perfluoroethylphenol
2-ethyl-4-perfluoropropylphenol
2-isopropyl-4-perfluoroethylphenol
2-tert-butyl-4-perfluoroethylphenol
2-(2-octyl)-4-perfluorobutylphenol
2,6-dimethyl-4-perfluoropentylphenol
2,6-diethyl-4-perfluoroethylphenol
2,6-diisopropyl-4-perfluoroisopropylphenol
2,6-di-tert-butyl-4-perfluoroethylphenol
2-tert-butyl-6-methyl-4-perfluorobutylphenol
2-methyl-4-perfluoromethylnaphthol
2-ethyl-4-perfluoromethylnaphthol
2-isopropyl-4-perfluoromethylnaphthol
2-tert-butyl-4-perfluoromethylnaphthol
2-(2-octyl)-4-perfluoromethylnaphthol
2-methyl-4-perfluoroethylnaphthol
2-ethyl-4-perfluoropropylnaphthol
2-isopropyl-4-perfluoroethylnaphthol
2-tert-butyl-4-perfluoroethylnaphthol
2-(2-octyl)-4-perfluorobutylnaphthol
2-benzyl-4-perfluoromethylnaphthol
2-cyclophentyl-4-perfluoromethylnaphthol
6-methyl-2-perfluoromethylphenol
6-ethyl-2-perfluoromethylphenol
6-isopropyl-2-perfluoromethylphenol
6-tert-butyl-2-perfluoromethylphenol
6-(2-decyl)-2-perfluoromethylphenol
6-benzyl-2-perfluoromethylphenol

6-cyclopentyl-2-perfluoromethylphenol
4,6-dimethyl-2-perfluoromethylphenol
4,6-diethyl-2-perfluoromethylphenol
4,6-diisopropyl-2-perfluoromethylphenol
4,6-di-tert-butyl-2-perfluoromethylphenol
4-tert-butyl-6-methyl-2-perfluoromethylphenol
4-benzyl-6-methyl-2-perfluoromethylphenol
4-cyclopentyl-6-ethyl-2-perfluoromethylphenol
4-ethyl-2-perfluoroethylphenol
4-ethyl-2-perfluoropropylphenol
4-isopropyl-2-perfluoroethylphenol
4-tert-butyl-2-perfluoroethylphenol
4-(2-dodecyl)-2-perfluorobutylphenol
4,6-dimethyl-2-perfluoropentylphenol
4,6-diethyl-2-perfluoroethylphenol
4,6-diisopropyl-2-perfluoroisopropylphenol
4,6-di-tert-butyl-2-perfluoroethylphenol
4-tert-butyl-6-methyl-2-perfluorobutylphenol

Ortho-hydrocarbyl perfluoroalkyl aromatic amines such as 2-alkyl- and 2,6-dialkyl-4-perfluoroalkyl anilines, 2-alkyl-4-perfluoroalkyl-1-naphthyl amines, and 6-alkyl- and 4,6-dialkyl-2-perfluoroalkyl anilines are useful as intermediates for the synthesis of crop protection chemicals such as herbicides and plant growth regulants and as intermediates for the synthesis of pesticides such as insecticides, miticides, acaricides, and fungicides.

Exemplary ortho-hydrocarbyl perfluoroalkyl aromatic amines include:
2-methyl-4-perfluoromethylaniline
2-ethyl-4-perfluoromethylaniline
2-isopropyl-4-perfluoromethylaniline
2-tert-butyl-4-perfluoromethylaniline
2-(2-octyl)-4-perfluoromethylaniline
2-benzyl-4-perfluoromethylaniline
2-cyclopentyl-4-perfluoromethylaniline
2,6-dimethyl-4-perfluoromethylaniline
2,6-diethyl-4-perfluoromethylaniline
2,6-diisopropyl-4-perfluoromethylaniline
2,6-di-tert-butyl-4-perfluoromethylaniline
2-tert-butyl-6-methyl-4-perfluoromethylaniline
2-benzyl-6-methyl-4-perfluoromethylaniline
2-cyclopentyl-6-ethyl-4-perfluoromethylaniline
2-ethyl-4-perfluoroethylaniline
2-ethyl-4-perfluoropropylaniline
2-isopropyl-4-perfluoroethylaniline
2-tert-butyl-4-perfluoroethylaniline
2-(2-octyl)-4-perfluorobutylaniline
2,6-dimethyl-4-perfluoropentylaniline
2,6-diethyl-4-perfluoroethylaniline
2,6-diisopropyl-4-perfluoroisopropylaniline
2,6-di-tert-butyl-4-perfluoroethylaniline
2-tert-butyl-6-methyl-4-perfluorobutylaniline
2-methyl-4-perfluoromethyl-1-naphthylamine
2-ethyl-4-perfluoromethyl-1-naphthylamine
2-isopropyl-4-perfluoromethyl-1-naphthylamine
2-tert-butyl-4-perfluoromethyl-1-naphthylamine
2-(2-octyl)-4-perfluoromethyl-1-naphthylamine

2-methyl-4-perfluoroethyl-1-naphthylamine
2-ethyl-4-perfluoropropyl-1-naphthylamine
2-isopropyl-4-perfluoroethyl-1-naphthylamine
2-tert-butyl-4-perfluoroethyl-1-naphthylamine
2-(2-octyl)-4-perfluorobutyl-1-naphthylamine
2-benzyl-4-perfluoromethyl-1-naphthylamine
2-cyclopentyl-4-perfluoromethyl-1-naphthylamine
6-methyl-2-perfluoromethylaniline
6-ethyl-2-perfluoromethylaniline
6-isopropyl-2-perfluoromethylaniline
6-tert-butyl-2-perfluoromethylaniline
6-(2-decyl)-2-perfluoromethylaniline
6-benzyl-2-perfluoromethylaniline
6-cyclopentyl-2-perfluoromethylaniline
4,6-dimethyl-2-perfluoromethylaniline
4,6-diethyl-2-perfluoromethylaniline
4,6-diisopropyl-2-perfluoromethylaniline
4,6-di-tert-butyl-2-perfluoromethylaniline
4-tert-butyl-6-methyl-2-perfluoromethylaniline
4-benzyl-6-methyl-2-perfluoromethylaniline
4-cyclopentyl-6-ethyl-2-perfluoromethylaniline
4-ethyl-2-perfluoroethylaniline
4-ethyl-2-perfluoropropylaniline
4-isopropyl-2-perfluoroethylaniline
4-tert-butyl-2-perfluoroethylaniline
4-(2-dodecyl)-2-perfluorobutylaniline
4,6-dimethyl-2-perfluoropentylaniline
4,6-diethyl-2-perfluoroethylaniline
4,6-diisopropyl-2-perfluoroisopropylaniline
4,6-di-tert-butyl-2-perfluoroethylaniline
4-tert-butyl-6-methyl-2-perfluorobutylaniline

Still other products which may be produced from the gem-dicyclohexadienones of this invention include (i) novel gem-disubstituted cyclohexadienones in which the gem-substitutents are a perfluoroalkyl group and a hydroxyl group, (ii) novel gem-disubstituted cyclohexanones in which the gem-substituents are a perfluoroalkyl group and a trihydrocarbylsiloxy group, (iii) novel gemdisubstituted cyclohexanols in which the gem-substituents are a perfluoroalkyl group and a trihydrocarbylsiloxy group, and (iv) novel gem-disubstituted cyclohexanones in which the gem-substituents are a perfluoroalkyl group and a hydroxyl group. Methods for effecting the synthesis of such compounds are illustrated in Examples XXX through XXXIX below.

## EXAMPLE XXX

### 4-Hydroxy-4-trifluoromethyl-2,5-cyclohexadien-1-one

A mixture of 200 mg (0.68 mmol) of 4-triethylsiloxy-4-trifluoromethyl-2,5-cyclohexadien-1-one and 1 mL of a solution of 1 part 37% hydrochloric acid in 9 parts absolute ethanol was heated at reflux overnight and poured into 10 mL of water. The resulting aqueous mixture was extracted with three 10 mL portions of dichloromethane. Combination, drying (MgSO$_4$), and concentration of the organic layers afforded a residue which was purified by PTLC (one 2 mm silica gel plate eluted with 1% methanol -99% dichloromethane) to give 109 mg (89% yield) of 4-hydroxy-4-trifluoromethyl-2,5-cyclohexadien-1-one. An analytical sample was obtained by crystallization from dichloromethane-hexane: mp 84-86° C; H NMR (CDCl$_3$) $\delta$ 3.40 (broad s, 1H), 6.40 (d, 2H, J = 10 Hz), 6.89 (d, 2H, J = 10 Hz); $^{13}$C NMR (CDCl$_3$) 70.2 (q, $J_{CF}$ = 30 Hz), 125.0 (q, $J_{CF}$ = 286 H), 132.2 (d), 142.7 (d), 184.5 (s) ppm; $^{19}$F NMR (CDCl$_3$) relative to CFCl$_3$) -79.6 ppm (s); IR (KBr) 3374, 3105, 3022, 2919, 1693, 1671, 1632, 1620, 1396, 1249, 1235, 1195, 1174, 1089, 1078, 1003, 988, 980, 973, 863, 698 cm$^{-1}$; mass spectrum (70 eV) m/z (relative intensity) 178 (5, M$^+$), 109 (100), 81 (34), 53 (36). Anal. Calcd. for C$_7$H$_5$F$_3$O$_2$: C, 47.20; H, 2.83. Found: C, 47.42; H, 2.80.

In Examples XXXI through XXXVI procedures as described in Example XXX were used.

## EXAMPLE XXXI

### 4-Hydroxy-4-trifluoromethyl-2,5-cyclohexadien-1-one

From 300 mg (0.80 mmol) of 4-tri-n-butylsiloxy-4-trifluoromethyl-2,5-cyclohexadien-1-one was obtained a product mixture. Gas chromatographic/mass spectral analysis showed that the major component of this mixture was 4-hydroxy-4-trifluoromethyl-2,5-cyclohexadien-1-one.

## EXAMPLE XXXII

### 2,6-Di-tert-butyl-4-hydroxy-4-trifluoromethyl-2,5-cyclohexadien-1-one

From 350 mg (0.87 mmol) of 2,6-di-tert-butyl-4-triethylsiloxy 4-trifluoromethyl-2,5-cyclohexadien-1-one was obtained a product mixture which was purified by PTLC (one mm silica gel plate eluted with dichloromethane) to give 245 mg (98% yield) of 2,6-di-tert-butyl-4-hydroxy-4-trifluoromethyl-2,5-cyclohexadien-1-one. An analytical sample was obtained by crystallization from hexane: mp 93-94° C; H NMR (CDCl₃) δ 1.25 (s, 18H), 2.57 (s, 1H), 6.48 (s, 2H); ¹³F NMR (CDCl₃, relative to CFCl₃) -79.8 to -79.9 ppm (m); IR (KBr) 3374, 3103, 3022, 2919, 1693, 1671, 1632, 1620, 1396, 1249, 1235, 1195, 1174, 1089, 1078, 1003, 988, 980, 973, 863, 698 cm⁻¹; mass spectrum (70 eV) m/z relative intensity) 290 (7, M⁺), 275 (18), 247 (20), 57 (100), 43 (35), 41 (62). Anal. Calcd. for C₁₅H₁₂F₃O₂: C, 62.05; H, 7.29. Found: C, 61.98; H, 7.46.

### EXAMPLE XXXIII

#### 4,6-Di-tert-butyl-2 hydroxy-2-trifluoromethyl-3,5-cyclohexadien-1-one

From 350 mg (0.87 mmol) of 4,6-di-tert-butyl-2-triethylsiloxy-2-trifluoromethyl-3,5-cyclohexadien-1-one was obtained a product mixture which was purified by PTLC (one 2 mm silica gel plate eluted with 20% dichloromethane 80% petroleum ether) to give 218 mg (87% yield) of 4,6-ditert-butyl-2-hydroxy-2-trifluoromethyl-3,5-cyclohexadien-1-one. An analytical sample was obtained by crystallization from hexane: mp 58-61° C; ¹H NMR (CDCl₃) δ 1.17 (s, 9H), 1.25 (s, 9H), 4.32 (s, 1H), 5.96 (d, 1H, J = 2Hz), 6.93 (d, 1H, J = 2Hz); ¹⁹F NMR (CDCl₃, relative to CFCl₃) -79.5 ppm (s); IR (KBr) 3456, 2964, 1676, 1372, 1367, 1254, 1234, 1217, 1186, 1163, 1151, 1124, 694 cm⁻¹; mass spectrum (70 eV) m/z (relative intensity) 290 (12, M⁺), 275 (20), 205 (28), 69 (30), 57 (100), 43 (27), 41 (77), 39 (20), Anal. Calcd. for C₁₅H₁₂F₃O₂: C, 62.05; H, 7.29. Found: C, 62.16; H, 7.27.

### EXAMPLE XXXIV

#### 9,10-Dihydro-10-hydroxy-9-oxo-10-trifluoromethylnaphthalene

From 350 mg (1.0 mmol) of 9,10-dihydro-9-oxo-10-triethylsiloxy-10-trifluoromethylnaphthalene was obtained a product mixture which was purified by PTLC (one 2 mm silica gel plate eluted with 1% methanol - 99% dichloromethane) to give 192 mg of 89% pure (73% yield) 9,10-dihydro-10-hydroxy-9-oxo-10-trifluoromethylnaphthalene. An analytical sample was obtained by crystallization from dichloromethane hexane: mp 73-76° C; ¹H NMR (CDCl₃) δ 3.94 (s, 1H), 6.48 (d, 1H, J = 10 Hz), 7.02 (d, 1H, J = 10 Hz), 7.43-8.18 (m, 4H); ¹⁹F NMR (CDCl₃, relative to CFCl₃) -80.0 ppm (s); IR-(KBr) 3368, 1667, 1627, 1597, 1454, 1377, 1301, 1283, 1230, 1188, 1172, 1156, 1142, 1102, 1047, 1017, 935, 838, 769, 754, 603, 560 cm⁻¹; mass spectrum (70 eV) m/z (relative intensity) 228 (8, M⁺), 159 (100), 131 (30), 103 (22), 77 (25). Anal. CalCd. for C₁₁H₇F₃O₂: C, 57.90; H, 3.09. Found: C, 57.94; H, 3.12.

### EXAMPLE XXXV

#### 9.10-Dihydro-10-hydroxy-9-oxo-10-trifluoromethylanthracene

From 340 mg (0.89 mmol) of 9,10-dihydro-9-oxo-10-triethylsilyolxy-10-trifluoromethylanthracene was obtained a product mixture which was purified by PTLC (one 2 mm silica gel plate eluted with dichloromethane) to give 223 mg (90% yield) of 9,10-dihydro-10-hydroxy-9-oxo-10-trifluoromethylanthracene. An analytical sample was obtained by crystallization from dichloromethane-hexane: mp 153-155° C; ¹H NMR (CDCl₃) δ 3.56 (s, IH), 7.47-7.82 (m, 4H), 7.92-8.31 (m, 4H); ¹⁹F NMR (CDCl₃, relative to CFCl₃) -79.8 ppm (s); IR (KBr) 3417, 1656, 1598, 1584, 1458, 1320, 1269, 1219, 1165, 1128, 1062, 933, 764, 716 cm⁻¹; mass spectrum (70 eV) m/z (relative intensity) 278 (1, M⁺), 209 (100), 152 (24). Anal. Calcd. for C₁₅H₉F₃O₂: C, 64.75; H, 3.26. Found: C, 64.63; H, 3.29.

### EXAMPLE XXXVI

#### 9,10-Dihydro-10-hydroxy-9-oxo-10-trifluoromethylphenanthrene

From 350 mg (0.89 mmol) of 9,10-dihydro-9-oxo-10-triethylsiloxy-10-trifluoromethylphenanthrene was obtained a product mixture that was purified by PTLC (one 2mm slica gel plate eluted with 50% dichloromethane - 50% petroleum ether) to give 238 mg (96% yield) of 9,10-dihydro-9-oxo-10-trifluoromethylphenanthrene. An analytical sample was obtained by crystallization from dichloro methane-hexane: mp 148-151° C; ¹H NMR (CDCl₃) δ 4.76 (s, 1H), 7.22-8.07 (m, 8H); ¹⁹F NMR (CDCl₃ relative to CFCl₃) -78.5 ppm (s); IR (KBr) 3455, 1687, 1598, 1479, 1451, 1321, 1299, 1286, 1228, 1210, 1167, 1110, 1056, 1015, 956, 941, 905, 778, 758, 731, 641, 615 cm⁻¹; mass spectrum (70 eV) m/z (relative intensity) 278 (31, M⁺), 209 (100), 181 (43), 152 (34), 75 (33). Anal. Calcd. for C₁₅H₉F₃O₂: C, 64.75; H, 3.26. Found: C, 64.75; H, 3.30.

## EXAMPLE XXXVII

### 4-Triethylsiloxy-4-trifluoromethylcyclohexanone

A solution of 291 mg of 4-triethylsiloxy-4-trifluoromethyl-2,5-cyclohexadien-1-one in 2 mL of absolute ethanol was treated with a few mg of 5% palladium on carbon, hydrogenated in a Parr shaker for one hour under 50 psig of hydrogen, and filtered. Concentration of the filtrate gave 4-triethylsiloxy-4-trifluoromethylcyclohexanone: ¹H NMR (CDCl₃) δ 0.45-1.16 (m, 15H), 1.92-2.89 (m, 8H); mass spectrum (70 eV) m/z relative intensity) 267 (19, M-C₂H₅), 115 (45), 105 (33), 87 (100), 81 (22), 77 (67), 73 (20), 67 (29), 59 (57), 55 (84), 47 (20).

## EXAMPLE XXXVIII

### 9,10-Dihydro-9-hydroxy-10-triethylsiloxy-10-trifluoromethylanthracene

A solution of 50 mg (0.13 mmol) of 9,10-dihydro-9-oxo-10-triethylsiloxy-10-trifluoromethylanthracene in 0.5 mL of absolute ethanol was treated successively with 42 mg (0.65 mmol) of zinc dust and 0.5 mL of a solution of 90% acetic acid - 20% water. The mixture was heated to reflux in a 110°C oil bath for 2 hours, allowed to cool to room temperature, and poured into 10 mL of water. The resulting aqueous mixture was extracted with three 5 mL portions of diethyl ether. Combination, drying, and concentration of the ether layers gave a residue which was purified by PTLC (one 1 mm silica gel plate eluted with 50% dichloromethane - 50% petroleum ether) to give 34 mg of 87% pure (58% yield) 9,10-dihydro-9-hydroxy-10-triethylsiloxy-10-trifluoromethylanthracene as a white solid: mass spectrum (70 eV) m/z (relative intensity) 363 (8, M-C₂H₅), 211 (100), 183 (21), 77 (25); TMS derivative 466 (M⁺), 368 (27), 246 (24), 196 (22), 193 (90), 165 (21), 105 (40), 87 (28), 77 (47), 73 (100), 59 (20), 45 (21).

## EXAMPLE XXXIX

### 4-Hydroxy-4-trifluoromethylcyclohexanone

A solution of 100 mg of 4-hydroxy-4-trifluoromethyl-2,5-cyclohexadien-1-one in 1 mL of absolute ethanol was treated with a few mg of 5% palladium on carbon, hydrogenated in a Parr shaker for one hour under 50 psig of hydrogen, and filtered. Gas chromatographic/mass spectral analysis indicated that the major component of the filtrate was 4-hydroxy-4-trifluoromethylcyclohexanone: mass spectrum (70 eV) m/z (relative intensity) 182 (11, M⁺), 55 (100), 42 (40).

## Claims

1. A gem-disubstituted cyclohexadienone in which the gem-substituents are a perfluoroalkyl group and a trihydrocarbylsiloxy group.

2. The cyclohexadienone of claim 1 wherein the perfluoroalkyl group is a trifluoromethyl group and the trihydrocarbylsiloxy group is a trialkylsiloxy group.

3. The cyclohexadienone of claim 2 wherein the gem substituents are in the para position relative to the keto group of the dienone.

4. The cyclohexadienone of claim 3 which is 4-triethylsiloxy-4-trifluoromethyl-2,5-cyclohexadien-1-one, 4-tributylsiloxy-4-trifluoromethyl-2,5-cyclohexadien-1-one, 2,6-di-tert-butyl-4-triethylsiloxy-4-trifluoromethyl-2,5-cyolohexadien-1-one, 1,4-dihydro-1-oxo-4-triethylsiloxy-4-trifluoromethylnaphthalene, or 9,10-dihydro-9-oxo-10-triethylsiloxy-10-trifluoromethylanthracene.

5. The cyclohexadienone of claim 2 wherein the gem substituents are in the ortho position relative to the keto group of the dienone.

6. The cyclohexadienone of claim 5 which is 4,6-di-tert-butyl-2-triethylsiloxy-2-trifluoromethyl-3,5- cyclohexadien-1-one or 9,10-dihydro-9-oxo-10-triethylsiloxy-10-trifluoromethylphenanthrene.

7. A process which comprises reacting a quinone with a perfluoroalkyltrihydrocarbylsilane under essentially anhydrous conditions in the presence of a catalyst as herein defined so that a gem-disubstituted cyclohexadienone is produced.

8. The process of claim 7 wherein the catalyst is potassium carbonate.

9. The process of claim 7 wherein the catalyst is an active quaternary ammonium bifluoride, quaternary phosphonium bifluoride, or alkali metal bifluoride.

10. The process of claim 7 wherein the catalyst is an alkali metal salt and is used together with a proton source.